Europäisches Patentamt

European Patent Office

Office européen des brevets

(11) Veröffentlichungsnummer: **0 280 754**
**A1**

## (12) EUROPÄISCHE PATENTANMELDUNG

(21) Anmeldenummer: 87103188.6

(51) Int. Cl.⁴: **A61M 15/02**

(22) Anmeldetag: 06.03.87

(43) Veröffentlichungstag der Anmeldung:
07.09.88 Patentblatt 88/36

(84) Benannte Vertragsstaaten:
**AT BE CH DE ES FR GB GR IT LI LU NL**

(71) Anmelder: **Schmidt, Klaus Dieter**
**Im Unteresch 21**
**D-8940 Memmingen(DE)**

(72) Erfinder: **Schmidt, Klaus Dieter**
**Im Unteresch 21**
**D-8940 Memmingen(DE)**

(74) Vertreter: **Sperling, Rüdiger, Dipl.-Ing. et al**
**Patentanwälte Dipl.Ing.S. Staeger**
**Dipl.Ing.Dipl.Wirtsch.Ing. R Sperling**
**Müllerstrasse 31**
**D-8000 München 5(DE)**

(54) **Heimklimagerät.**

(57) Ein Heimklimagerät weist an seinem Lufteintritt (27) eine Filteranordnung, bestehend aus einem Vorfilter (2) und Aktivkohlefilterschichten (3) auf, die in einer außerhalb des Gehäuses angeordneten, von außen zugänglichen Filterkassette (24) eingesetzt sind. Des weiteren ist eine Ionenaufladevorrichtung (7) vorgesehen, die die das Gerät durchströmende Luft mit Ionen anreichert. Eine Befeuchtungseinrichtung dient der Anreicherung der Luft mit Feuchtigkeit, wobei zusätzlich nach der Befeuchtungseinrichtung eine weitere Ionenaufladestation vorgesehen sein kann (7'). An die Luftaustrittsöffnung (28) ist eine Adapterkassette mit angeschlossenem Inhalationschlauch (3a) und Inhaliermaske (4a) vorgesehen, wobei in die Adapterkassette nach Wahl ein mit Zusatzstoffen getränktes Vlies (1a) einlegbar ist.

Fig. 1

# HEIMKLIMAGERÄT

Die Erfindung betrifft ein elektromedizinisches Heimklimagerät, das sowohl als Raumklimagerät als auch als medizinisches Inhaliergerät angewendet werden kann. Es weist ein teilbares Gehäuse mit einem Flüssigkeitsbehälter, einer Lufteintritts- und einer Luftaustrittsöffnung, eine an der Lufteintrittsöffnung vorgesehene Filteranordnung, ein Gebläse und eine Befeuchtungseinrichtung auf, die mittels einer Pumpe mit Flüssigkeit aus dem Flüssigkeitsbehälter versorgt wird, wobei der Flüssigkeitsbehälter im unteren Bereich des Gehäuses im wesentlichen durch eine Trennwand von einem oberen Luftwegbereich abgeschlossen ist. Bei einem bekannten Heimklimagerät ist das Auswechseln des Eingangsfilters nur nach Entfernen der oberen Gehäuseabdeckung möglich. Dieses bekannte Heimklimagerät besitzt darüber hinaus eine Einrichtung zum Bestrahlen der Luft mit UV-Strahlung verschiedenster Kategorien. Die Befeuchtungseinrichtung ist so ausgelegt, daß das aus der Tropfeinrichtung austretende Wasser nach Durchlaufen eines Kräuternetzes in einen Auffangtrichter tropft.

Diese Vorrichtung weist den Nachteil auf, daß das abtropfende Wasser ständig ein Tropfgeräusch verursacht, das im Laufe der Zeit als störend empfunden wird.

Die UV-Strahlung ist zwar bedingt in der Lage, eine gewisse Keimabtötung zu bewirken, eine unmittelbare Beeinflussung der Luft ergibt sich daraus jedoch nicht.

Der Erfindung liegt die Aufgabe zugrunde, ein Heimklimagerät der genannten Gattung zu schaffen, mit dem es möglich ist, ein Luftklima zu schaffen, das vergleichbar ist mit dem Luftklima nach einem Gewitter.

Die Aufgabe wird erfindungsgemäß dadurch gelöst, daß die Filteranordnung aus einem Vorfilter und Aktivkohlefilterschichten besteht und in einer außerhalb des Gehäuses angeordneten, von außen zugänglichen Filterkassette eingesetzt ist und daß eine den gefilterten Luftstrom ionisierende Ionenaufladevorrichtung vorgesehen ist.

In der Radioästhesie ist es bekannt, daß ein Luftklima, das eine bestimmte Luftfeuchtigkeit sowie einen Überschuß an Negativionen besitzt, eine positive Wirkung auf den menschlichen Organismus ausüben. Das erfindungsgemäße Heimklimagerät ermöglicht durch die besondere Filteranordnung eine hochgradige Reinigung der eintretenden Luft, die anschließend mittels einer Ionenaufladestation eine Negativionisation erfährt.

Eine vorteilhafte Ausbildung kann darin gesehen werden, daß das Gebläse unmittelbar an der Lufteintrittsöffnung angeordnet ist. Auf diese Weise wird gewährleistet, daß die Luft ausschließlich durch die Lufteintrittsöffnung angesogen wird und somit durch die Filteranordnung in das Gerät eintreten muß.

Einen günstigen Wirkungsgrad bei der Anreicherung des Luftstroms mit Negativionen kann man dadurch erzielen, daß die Ionenaufladevorrichtung eine unmittelbar hinter dem Gebläse angeordnete Gitterkonstruktion aufweist, die negativ gepolt ist. Die gereinigte Luft strömt über die als Sprühelektrode wirkenden Gitterstäbe und trägt Elektronen mit sich.

Vorteilhaft kann es sein, daß die Ionenaufladevorrichtung zwischen der Befeuchtungseinrichtung und dem Luftaustritt vorgesehen ist, wobei eine besonders günstige Ausbildung darin gesehen werden kann, daß sowohl hinter dem Gebläse als auch unmittelbar hinter der Befeuchtungseinrichtung die Ionisation durchgeführt wird. Auf diese Weise erhält man die besten Ionisationsergebnisse.

Aus Sicherheitsgründen kann vorgesehen sein, daß die Gitterkonstruktionen der Ionenaufladevorrichtungen eine derartige Schaltung aufweisen, daß beim Öffnen des Gehäuses der Stromkontakt unterbrochen und die Ladung zur Erde abgeleitet wird.

Bei einer weiteren vorteilhaften Ausgestaltung kann vorgesehen sein, daß, wenn die überschüssige, von der Befeuchtungseinrichtung ausgetragene Flüssigkeit über eine Filteranordnung in den Flüssigkeitsbehälter zurückgeführt wird, diese Filteranordnung aus einem oberhalb des Flüssigkeitspegels angeordneten, unmittelbar der überschüssigen Flüssigkeit ausgesetzten austauschbaren Auffangvlies besteht, unter dem ein Rücklauffilter angeordnet ist, wobei das Auffangvlies und das Rücklauffilter strömungsverbunden sind. Dabei ist es günstig, daß das Auffangvlies in einer wannenartigen Vertiefung der Trennwand liegt. Hierbei ist es vorteilhaft, daß der Pegel der Flüssigkeit in dem Flüssigkeitsbehälter unterhalb der Wanne liegt.

Bei einer Befeuchtungseinrichtung, die die Flüssigkeit in Form eines Sprühnebels oder eines Tropfenvorhangs abgibt, kann gemäß einer günstigen Ausbildung vorgesehen werden, die Düsenanordnung an einer Wand eines Druckbehälters auszubilden, der von der Pumpe über ein Zuführrohr versorgt wird. Dieser Druckbehälter hat eine gewisse Speicherkapazität, so daß Schwankungen im Pumpenbetrieb ohne Auswirkungen auf den Feuchtigkeitsgehalt ausgeglichen werden können. Auch wird auf diese Weise sichergestellt, daß unmittelbar nach Starten des Geräts der gewünschte Feuchtigkeitsgehalt in der Luft vorhanden ist.

Gemäß einer vorteilhaften Weiterbildung kann dann, wenn eine Inhaliermaske über einen Schlauch mit der Luftaustrittsöffnung verbunden ist, vorgesehen sein, daß der Schlauch an seinem geräteseitigen Ende eine Adapterkassette mit einem Vlies zur Aufnahme von Zusatzstoffen aufweist. Das Vlies wird dann mit den Zusatzstoffen getränkt und gibt diese Zusatzstoffe an die an ihm vorbeistreichende befeuchtete und ionisierte Luft ab.

Dabei ist es vorteilhaft, wenn das Vlies in einer wannenartigen Vertiefung aufgenommen und von außen zugänglich ist. Auf diese Weise kann das Vlies leicht ausgetauscht oder Zusatzstoff nachgefüllt werden, ohne daß der Adapter von der Öffnung abgebaut werden muß.

Bei einer abgewandelten Ausführungsform ist vorgesehen, daß das Vlies sich zumindest teilweise über den Querschnitt des Luftdurchtritts in der Kassette erstreckt. Auf diese Weise ist eine maximale Aufnahme von Zusatzstoffen gewährleistet, wobei das Vlies eine ausreichende Luftdurchlässigkeit aufzuweisen hat.

Im folgenden wird die Erfindung anhand eines in der Zeichnung dargestellten Ausführungsbeispiels näher erläutert. Es zeigen:

Fig. 1 eine Ansicht des erfindungsgemäßen Heimklimageräts im Schnitt, und

Fig. 2 eine Seitenansicht des erfindungsgemäßen Heimklimageräts mit Inhaliermaske, Zuführschlauch und Adapter, letzterer teilweise aufgebrochen.

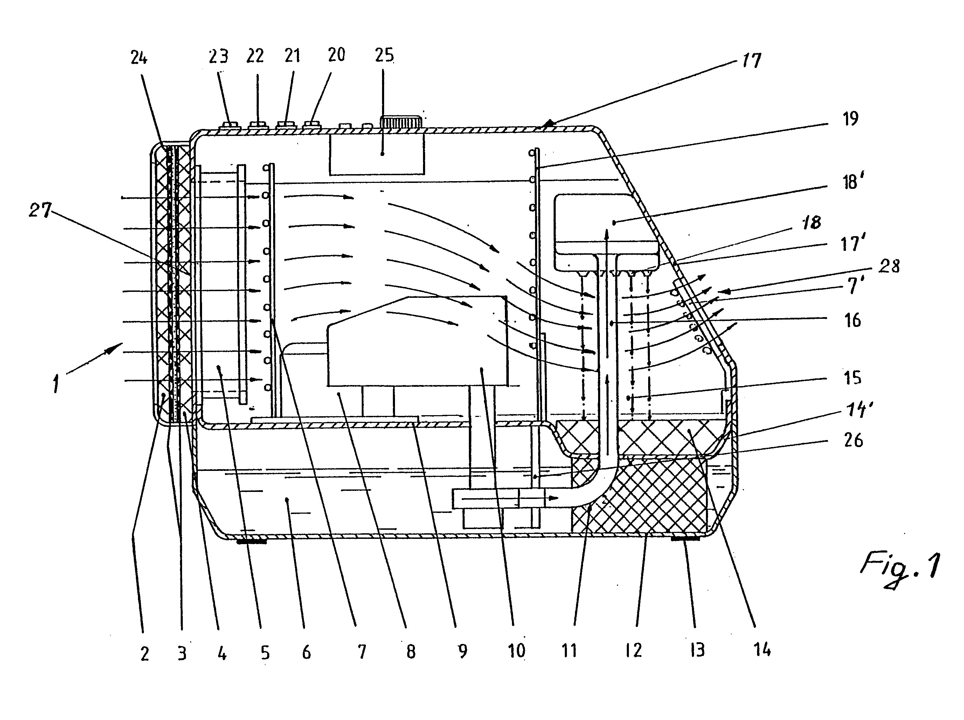

In Fig. 1 ist ein erfindungsgemäßes Heimklimagerät im Längsschnitt dargestellt. Das Heimklimagerät weist ein im wesentlichen horizontal geteiltes Gehäuse 17. mit einem Gehäuseoberteil 17' und einem Gehäuseunterteil 12 auf. Das Gehäuseunterteil ist als Flüssigkeitsbehälter ausgebildet.

Das Gehäuseoberteil 17' besitzt eine Lufteintrittsöffnung 27 und eine Luftaustrittsöffnung 28. An der Gehäuseaußenseite ist vor der Lufteintrittsöffnung ein Filtergehäuse 24 angeordnet, in dem eine Filteranordnung, bestehend mindestens aus einem Vorfilter 2 und Aktivkohlefilterschichten 3 eingesetzt sind. Es kann zusätzlich ein feiner Vlies 4 zum Aussondern letzter Staubpartikel als letzte Filterlage unmittelbar vor der Öffnung vorgesehen sein. Das Filtergehäuse ist von außen zugänglich, so daß die Filter, insgesamt oder teilweise, einfach auswechselbar sind.

Unmittelbar hinter der Lufteintrittsöffnung 27 ist ein Gebläse 5 angeordnet, das die Luft durch die Filterschichten ansaugt und zur weiteren Aufbereitung in das Gehäuseinnere einbläst.

In dem Gehäuseinneren sind zur Ionisierung der Luft, insbesondere der Sauerstoffpartikel zumindest eine Ionenaufladevorrichtung angeordnet.

Eine solche Ionenaufladevorrichtung ist in der dargestellten Ausführungsform unmittelbar hinter dem Gebläse 5 angeordnet. Die Ionenaufladevorrichtung besteht aus einer Gitterkonstruktion 7, die in dem aus dem Gebläse 5 austretenden Luftstrom eingesetzt ist. Ein weiteres Ionisationsgitter kann unmittelbar vor dem Luftaustritt zur Ionisierung der befeuchteten Luft angebracht sein. Die Ionisationsgitter bestehen im wesentlichen aus seitlichen Rahmenelektroden, zwischen denen Emissionsdrähte gespannt sind, die nach Art einer Sprühelektrode Elektronen abgeben. Obwohl in der Zeichnung nicht dargestellt, befinden sich diese Ionisationsgitter jeweils in einer Schutzgitterkonstruktion, durch welche ein Berühren der eigentlichen Elektrode verhindert wird.

Aus Sicherheitsgründen ist die Schaltung der Ionenaufladevorrichtung so ausgelegt, daß bei einem Abnehmen des oberen Gehäuseteils der Stromfluß, sollte er nicht vorher durch Betätigen eines entsprechenden Knopfes ausgeschaltet worden sein, unterbrochen wird. Des weiteren sind Kontakte an den Ionengitterkonstruktionen vorgesehen, die an Erde gelegt werden, wenn das Gehäuseoberteil abgenommen wird.

Die Befeuchtungseinrichtung besteht im dargestellten Ausführungsbeispiel aus einer Abtropfdusche, die über ein Zufuhrrohr und mit Hilfe einer Pumpe mit Flüssigkeit gespeist wird. Die Abtropfdusche ist als eine Düsenanordnung an der Bodenwand eines Druckbehälters 18 ausgebildet, in den das Zuführrohr 16 einmündet. Die aus der Düsenanordnung austretende Flüssigkeit wird am Boden des Luftkanals von einem Auffangvlies 14 aufgenommen, das in einer wannenartigen Vertiefung 14' der Trennwand 9 eingelegt ist. Die wannenartige Vertiefung 14' weist eine Vielzahl von Öffnungen auf, durch die die Flüssigkeit zu einem unmittelbar unter der Vertiefung angeordneten Rücklauffilter 11 laufen kann, das in dem Behälter angeordnet ist und aus einem geeigneten Schaumstoffmaterial bestehen kann.

Wesentlich ist, daß der höchste Pegel in dem Flüssigkeitsbehälter niedriger liegt als das Auffangvlies, so daß die Flüssigkeit sich nicht in dem Auffangvlies ansammeln kann, sondern zügig abfließt. In Strömungsrichtung der Luft vor der Befeuchtungseinrichtung ist bei dem dargestellten Ausführungsbeispiel eine Schutzgitterkonstruktion vorgesehen, die eine bauliche Einheit mit den Schutzgitteraufbauten bildet, die die Ionenaufladevorrichtung gegen Zugriff von außen abschirmen.

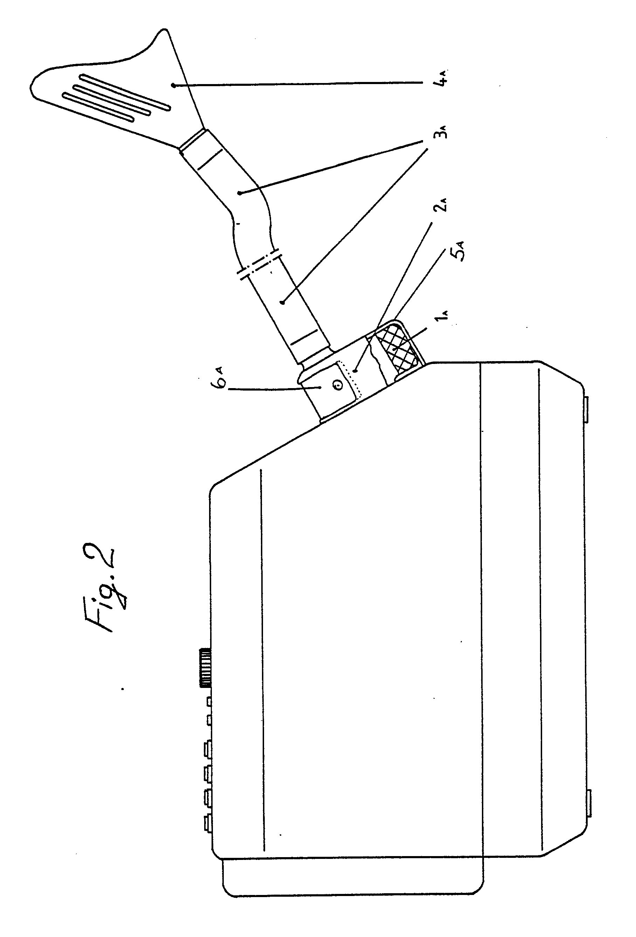

In Fig. 2 ist eine Inhalationsvorrichtung an dem Klimagerät angeschlossen, die aus einer Inhaliermaske 4a, einem Schlauch 3a und einer Adapterkassette besteht, wobei die Adapterkassette unmittelbar an die Luftaustrittsöffnung des Geräts angebracht wird. In der Adapterkassette ist ein Vlies 1a zur Aufnahme von Zusatzstoffen eingelegt. Das Vlies und die als Inhalationszusatz vorgesehenen Essenzen können bei an dem Gerät befestigten Adapter leicht durch die verschließbare Öffnung 6a ergänzt oder ausgetauscht werden. Der Verschluß kann dabei aus einem Klappdeckel oder einem verschiebbaren Wandbereich bestehen.

Das Vlies ist in einer wannenartigen Vertiefung aufgenommen, so daß eine optimale Sättigung erzielt werden kann. Das Vlies kann darüber hinaus so ausgebildet sein, daß es sich mit einem Teilabschnitt ganz oder teilweise über den Querschnitt des Luftdurchtritts der Kassette erstreckt. In einem solchen Fall ist das Vlies derart porös und luftdurchlässig ausgebildet, daß eine ausreichende Luftzufuhr ermöglicht wird. Eine solche Ausbildung gestattet eine maximale An reicherung der durchströmenden Luft mit dem Zusatzstoff.

Zum Betätigen der verschiedenen Einheiten im Gerät sind ein Schalter 20 für die Flüssigkeitspumpe, ein Schalter 21 für den Lüfter und ein Schalter 22 für die Ionenaufladung vorgesehen. Ein Hauptschalter 23 ist über einen Schaltkreis so mit allen Elementen geschaltet, daß sowohl die Pumpe, der Lüfter und der Ionentauscher in vorprogrammierter Weise in Tätigkeit gesetzt werden können. Dieser Hauptschalter kann mit einer Zeitschaltuhr 25 gekoppelt sein, die den Betrieb des Geräts nach einer eingestellten Zeit unterbricht.

Schließlich ist eine von außen ablesbare Flüssigkeitsstandsanzeige im Flüssigkeitsbehälter angeordnet.

**Ansprüche**

1. Heimklimagerät mit mindestens einem in einem teilbaren Gehäuse vorgesehenen Flüssigkeitsbehälter, einer Lufteintritts-und einer Luftaustrittsöffnung, einer an der Lufteintrittsöffnung vorgesehenen Filteranordnung, einem Gebläse und einer Befeuchtungseinrichtung, die mittels einer Pumpe mit Flüssigkeit aus dem Flüssigkeitsbehälter versorgt wird, wobei der Flüssigkeitsbehälter im unteren Bereich des Gehäuses im wesentlichen durch eine Trennwand von einem oberen Luftwegbereich abgeschlossen ist, dadurch **gekennzeichnet**, daß die Filteranordnung (1) aus einem Vorfilter (2) und Aktivkohlefilterschichten (3) besteht und in einer außerhalb des Gehäuses angeordneten, von außen zugänglichen Filterkassette (24) eingesetzt ist, und daß eine den gefilterten Luftstrom ionisierende Ionenaufladevorrichtung (7, 8) vorgesehen ist.

2. Heimklimagerät nach Anspruch 1, dadurch **gekennzeichnet**, daß das Gebläse (5) unmittelbar an der Lufteintrittsöffnung angeordnet ist.

3. Heimklimagerät nach Anspruch 1 oder 2, dadurch **gekennzeichnet**, daß die Ionenaufladevorrichtung eine unmittelbar hinter dem Gebläse angeordnete Gitterkonstruktion (7) aufweist.

4. Heimklimagerät nach mindestens einem der Ansprüche 1 bis 4, dadurch **gekennzeichnet**, daß die Ionenauf ladevorrichtung (7, 8) zwischen der Befeuchtungseinrichtung (18) und dem Luftaustritt vorgesehen ist.

5. Heimklimagerät nach mindestens einem der Ansprüche 3 bis 5, **gekennzeichnet** durch eine Kombination der Anordnungen gemäß den Ansprüchen 4 und 5.

6. Heimklimagerät nach mindestens einem der Ansprüche 3 bis 5, dadurch **gekennzeichnet**, daß die Gitterkonstruktionen der Ionenaufladevorrichtungen (7m 7', 8) eine derartige Schaltung aufweisen, daß beim Öffnen des Gehäuses der Stromkontakt unterbrochen und die Ladung zur Erde abgeleitet wird.

7. Heimklimagerät nach mindestens einem der Ansprüche 1 bis 6, dadurch gekennzeichnet, daß den Aktivkohlefilterschichten (3) ein Feinfilter (4) nachgeordnet ist.

8. Heimklimagerät nach mindestens einem der Ansprüche 1 bis 7, wobei die von der Befeuchtungseinrichtung ausgetragene überschüssige Flüssigkeit über eine Filteranordnung in den Flüssigkeitsbehälter zurückgeführt wird, dadurch **gekennzeichnet**, daß die Filteranordnung (11, 14) aus einem oberhalb des Flüssigkeitspegels angeordneten, unmittelbar der überschüssigen Flüssigkeit ausgesetztem austauschbarem Auffangvlies (14) und einem darunter angeordneten, mit dem Auffangvlies in Strömungsverbindung stehenden Rücklauffilter (11) besteht.

9. Heimklimagerät nach Anspruch 8, dadurch **gekennzeichnet**, daß das Auffangvlies (14) in einer wannenartigen Vertiefung (14') der Trennwand (9) liegt.

10. Heimklimagerät nach mindestens einem der Ansprüche 1 bis 8, wobei die Befeuchtungseinrichtung eine die Flüssigkeit in den Luftstrom abgebende Sprüh-oder Tropfdüsenanordnung aufweist, dadurch **gekennzeichnet**, daß die Düsenanordnung (18) an einer Wand eines Druckbehälters (18') ausgebildet ist, der von der Pumpe (10) über ein Zuführrohr (16) versorgt wird.

11. Heimklimagerät nach mindestens einem der Ansprüche 1 bis 10, mit einem an der Luftaustrittsöffnung anbringbaren Schlauch, der an seinem freien Ende in eine Inhaliermaske einmündet, dadurch **gekennzeichnet**, daß der Schlauch (3a) an seinem geräteseitigen Ende eine Adapterkassette (2a) mit einem Vlies (1a) zur Aufnahme von Zusatzstoffen aufweist.

12. Heimklimagerät nach Anspruch 11, dadurch **gekennzeichnet,** daß das Vlies (1a) in einer wannenartigen Vertiefung aufgenommen und von außen zugänglich ist.

13. Heimklimagerät nach Anspruch 11 oder 12, dadurch **gekennzeichnet,** daß das Vlies (1a) sich zumindest teilweise über den Querschnitt des Luftdurchtritts in der Kassette (2a) erstreckt.

0 280 754

24  23 22 21 20  25                                  17

                                                     19

27                                                   18'

                                                     18
                                                     17'
1                                                    28
                                                     7'

                                                     16

                                                     15

                                                     14'

                                                     26

2  3  4  5  6  7  8  9  10  11  12  13  14

*Fig.1*

Fig.2

6ᴀ

1ᴀ 5ᴀ 2ᴀ 3ᴀ 4ᴀ

0 280 754

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-1 555 598 (R.F. FIGEAT) * Seite 1, rechte Spalte, Zeilen 5,6,15,16,23-25,27; Seite 2, linke Spalte, Zeilen 34-36 * | 1 | A 61 M 15/02 |
| | --- | | |
| A | FR-A-2 276 840 (P. GUICHARD) * Seite 5, Zeilen 19,20; Seite 6, Zeile 18; Seite 8, Zeilen 8-12; Seite 12, Zeilen 16-22 * | 1 | |
| | ----- | | |

**RECHERCHIERTE SACHGEBIETE (Int. Cl.4)**

A 61 M

Der vorliegende Recherchenbericht wurde für alle Patentansprüche erstellt.

| Recherchenort | Abschlußdatum der Recherche | Prüfer |
|---|---|---|
| DEN HAAG | 06-11-1987 | GERARD B.E. |